# EUROPÄISCHE PATENTSCHRIFT

(11) **EP 3 009 250 B1**
(45) Veröffentlichungstag und Bekanntmachung des Hinweises auf die Patenterteilung: **08.04.2020**
(21) Anmeldenummer: 15184877.7
(22) Anmeldetag: 11.09.2015
(51) Int. Cl.: B29C 45/16, A61M 39/26, B29C 45/00, B29C 35/08, B29K 83/00, B29L 31/00

(54) **KUNSTSTOFFSPRITZGUSSWERKZEUG ZUR HERSTELLUNG EINES MEHRKOMPONENTENSPRITZGUSSBAUTEILS**
PLASTIC INJECTION MOULDING TOOL FOR MANUFACTURING A MULTI-COMPONENT INJECTION MOULDING PART
OUTIL DE MOULAGE PAR INJECTION EN PLASTIQUE DESTINE A FABRIQUER UN COMPOSANT DE MOULAGE PAR MULTI-INJECTION DE PLUSIEURS COMPOSANTS

(30) Priorität: 17.10.2014 DE 102014221158
(43) Veröffentlichungstag der Anmeldung: 20.04.2016
(73) Patentinhaber: B. Braun Melsungen AG, 34212 Melsungen (DE)
(72) Erfinder: SCHLITT, Christof, 34621 Obergrenzebach (DE)
(74) Vertreter: Patentanwälte Ruff, Wilhelm, Beier, Dauster & Partner mbB

(56) Entgegenhaltungen:
- WO-A1-2013/186185
- DE-A1-102004 053 214
- DE-A1-102010 002 141

## Beschreibung

Die Erfindung betrifft ein Kunststoffspritzgusswerkzeug nach dem Oberbegriff des Anspruchs 1.

Die DE 10 2004 053 214 A1 offenbart ein Rückschlagventil für die Medizintechnik, das ein zweiteiliges Schlauchanschlussgehäuse aufweist. Ein Abschnitt des einen Gehäuseteils des Schlauchanschlussgehäuses ist von einer aus einem Elastomer bestehenden Membran umspritzt. Nach dem Umspritzen der Membran und demzufolge der Verbindung der Membran mit dem einen Gehäuseteil wird dieses Gehäuseteil mit dem anderen Gehäuseteil des Schlauchanschlussgehäuses zusammengefügt. Es ergeben sich zwei harte Bauteilkomponenten, die zusammengefügt werden. Einer harten Bauteilkomponente ist die elastisch flexible Membran zugeordnet. Der eine Gehäuseteil dient demzufolge als Träger für die flexible Membran. Der andere Gehäuseteil dient dazu, die Membran zu umschließen.

Aus der WO 2013/186185 A1 ist ein Verfahren zum Herstellen eines Verbundformkörpers mit einer Hartkomponente und einer Weichkomponente bekannt. Ein solcher Verbundformkörper kann als Intravenös-Ventil in der Medizintechnik eingesetzt werden. Details über eine Gestaltung eines Kunststoffspritzgusswerkzeugs zur Herstellung dieses Verbundformkörpers sind nicht offenbart.

Mehrkomponentenspritzgussbauteile aus Kunststoff, die eine harte Bauteilkomponente aus einem thermoplastischen Kunststoff sowie eine weiche Bauteilkomponente aus einem Elastomermaterial aufweisen, sind allgemein bekannt. Derartige Mehrkomponentenspritzgussbauteile werden in einem Kunststoffspritzgusswerkzeug hergestellt, dessen Werkzeugform sowohl eine thermoplastische Kunststoffkomponente als auch eine Elastomerkomponente in fließfähigem Zustand zugeführt werden können. Da die Elastomerkomponente bei relativ hohen Temperaturen aushärtet, ist es notwendig, für die thermoplastische Kunststoffkomponente eine noch höhere Schmelztemperatur vorzusehen, um sicherzustellen, dass die in einem ersten Verfahrensschritt bereits zugeführte und bis zur Erstarrung abgekühlte thermoplastische Kunststoffkomponente beim Aushärten der Elastomerkomponente in der Werkzeugform nicht wieder aufschmilzt. Für die Herstellung derartiger Mehrkomponentenspritzgussbauteile können daher nur hochwertige und teure Thermoplaste eingesetzt werden mit Schmelztemperaturen, die oberhalb der Aushärtetemperatur der entsprechenden Elastomerkomponente liegen.

Aufgabe der Erfindung ist es, ein Kunststoffspritzgusswerkzeug der eingangs genannten Art zu schaffen, das eine einfache und kostengünstige Herstellung eines Mehrkomponentenspritzgussbauteils ermöglicht.

Diese Aufgabe wird durch die Merkmale des Anspruchs 1 gelöst. Die UV-Lichtquelle ist entsprechenden Kavitäten der Werkzeugform derart zugeordnet, dass sich nach dem Einbringen der UV-härtenden Elastomerkomponente in entsprechende Kavitäten der Werkzeugform eine gleichmäßige UV-Bestrahlung dieser Kavitäten ergibt. Erfindungsgemäß ist die Werkzeugform zur Herstellung eines zuvor beschriebenen Ventilbauteils für den Einsatz bei medizinischen Infusionssystemkomponenten gestaltet. Das Kunststoffspritzgusswerkzeug kann mehrere, identisch gestaltete Werkzeugformen umfassen, um eine gleichzeitige Herstellung mehrerer dieser Ventilbauteile zu ermöglichen. Aufgrund der UV-Aushärtung des Elastomermaterials entsteht bei der Herstellung des Mehrkomponentenspritzgussbauteils keine große Wärmeeinwirkung aufgrund der UV-Aushärtung des Elastomermaterials. Dadurch ist es möglich, nahezu alle Arten von thermoplastischen Kunststoffen zur Herstellung der harten Bauteilkomponenten einzusetzen. Die Verbindung zwischen der weichen Bauteilkomponente und der harten Bauteilkomponente erfolgt während des Herstellvorgangs in einem entsprechenden Kunststoffspritzgusswerkzeug. In besonders vorteilhafter Weise wird als UV-härtendes Elastomermaterial UV-härtendes Silikon eingesetzt.

Erfindungsgemäß ist das Mehrkomponentenspritzgussbauteil als Ventilbauteil für den Einsatz bei medizinischen Infusionssystemkomponenten gestaltet. Dadurch, dass für die Herstellung der weichen Bauteilkomponente nur wenig Wärmeeinwirkung aufgrund der UV-Aushärtung notwendig ist, ergibt sich für die weiche Bauteilkomponente bei der Herstellung nahezu kein Schrumpf. Dies ermöglicht eine Minimierung des Materialeinsatzes bezüglich des UV-härtenden Elastomermaterials. Zudem kann das Ventilbauteil eine besonders gute Funktionalität aufweisen, insbesondere im Hinblick auf Dichtigkeitsanforderungen oder Öffnungsdrücke des Ventilbauteils.

Erfindungsgemäß ist das Ventilbauteil mit einem formstabilen Gehäusekörper aus einem thermoplastischen Kunststoff sowie mit einer elastisch flexiblen Ventilkomponente versehen, die aus dem UV-härtenden Elastomermaterial hergestellt ist. Die Ventilkomponente ist vorzugsweise aus einem UV-härtenden Silikon hergestellt. Die elastische Ventilkomponente ist vorzugsweise als Ventilmembran ausgeführt, kann aber bei anderen Varianten der Erfindung auch in anderen Ausgestaltungsformen, wie insbesondere einer Schnabel- oder Kuppelgeometrie, vorgesehen sein.

Vorteilhaft ist der Gehäusekörper aus einem thermoplastischen Kunststoff mit einer Schmelztemperatur unter 180 °C, insbesondere aus Polyolefinen, ABS, PS, PC, hergestellt. Dadurch ist es möglich, für die Herstellung des Gehäusekörpers nahezu alle üblichen Standard-Thermoplaste einzusetzen, wodurch das entsprechende Ventilbauteil in der Herstellung wesentlich günstiger wird als beim Stand der Technik. Dadurch, dass das Ventilbauteil einschließlich Gehäusekörper und elastisch flexibler Ventilmembran einstückig hergestellt ist, wird zum einen eine Herstellung in großen Stückzahlen in wirtschaftlich sinnvoller Art ermöglicht. Zum anderen entfällt ein zusätzlicher Montageaufwand zum Zusammenfügen von Gehäusekörper und Ventilmembran, wie dies beim Stand der Technik der Fall ist.

In Ausgestaltung des Kunststoffspritzgusswerkzeugs umfasst die UV-Lichtquelle eine LED-Anordnung mit einem UV-Strahlungsbereich. Dies ist eine besonders energiesparende Variante einer UV-Lichtquelle, die zudem platzsparend im Bereich der Werkzeugform unterbringbar ist.

In weiterer Ausgestaltung der Erfindung umfasst die UV-Lichtquelle eine Lichtleiteranordnung, insbesondere zur Leitung der UV-Lichtstrahlung der LED-Anordnung. Die Lichtleiteranordnung ermöglicht eine besonders gleichmäßige UV-Bestrahlung der entsprechenden Kavitäten, da sie entlang der Kavitäten verlegt sein kann.

Weitere Vorteile und Merkmale der Erfindung ergeben sich aus den Ansprüchen sowie aus der nachfolgenden Beschreibung eines bevorzugten Ausführungsbeispiels der Erfindung, das anhand der Zeichnungen dargestellt ist.
- Fig. 1: zeigt schematisch einen Längsschnitt durch einen Teil einer Tropfkammer eines medizi-nischen Infusionssystems mit einer Ausführungsform eines Mehrkomponentenspritz-gussbauteils in Form eines Ventilbauteils,
- Fig. 2: das Ventilbauteil nach Fig. 1 in vergrößerter Schnittdarstellung und
- Fig. 3: schematisch einen Querschnitt durch eine Werkzeugform einer Ausführungsform eines erfindungsgemäßen Kunststoffspritzgusswerkzeugs.

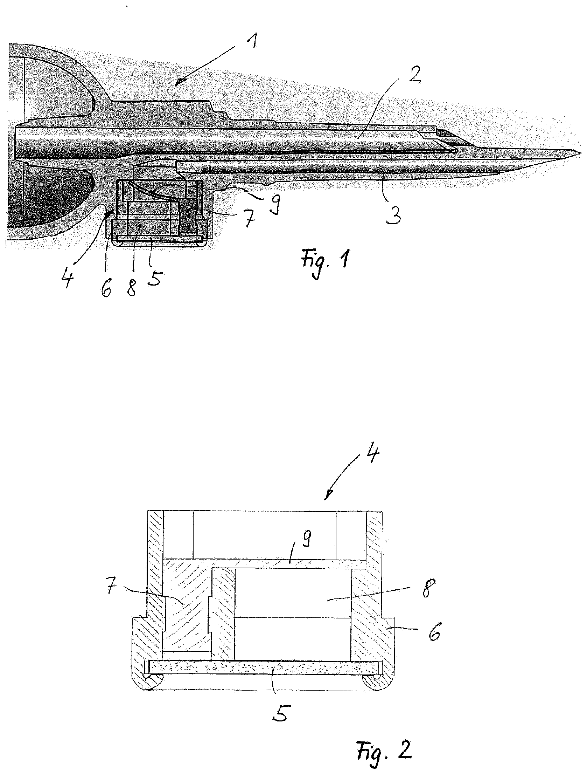

Eine Tropfkammer 1 für ein medizinisches Infusionssystem weist im Bereich einer Einstechspitze einen Fluidführungskanal 2 auf, dem ein parallel verlaufender Belüftungskanal 3 zugeordnet ist. Der Belüftungskanal 3 ist zur Umgebung hin offen über ein Ventilbauteil 4, das in einem Trägergehäuse der Tropfkammer 1 eingesetzt ist. Das Ventilbauteil 4 weist einen Gehäusekörper 6 auf, der einen sowohl zum Belüftungskanal 3 als auch zur Umgebung nach außen hin offenen Durchtritt 8 umschließt. In dem Gehäusekörper 6 ist eine elastisch flexible Ventilmembran 9 vorgesehen, die eine Funktion eines Rückschlagventils besitzt. Die Ventilmembran 9 geht einstückig in ein Trägerelement 7 über, das form- und stoffschlüssig in dem Gehäusekörper 6 gehalten ist. Dem Durchtritt 8 ist im Bereich seiner zur Umgebung hin offenen Austrittsöffnung ein Bakterienfilter 5 zugeordnet, der zur Reinigung der aus der Umgebung in den Belüftungskanal 3 strömenden Luft dient. Die elastisch flexible Ventilmembran 9 öffnet gemäß der Darstellung nach Fig. 1 nach innen zum Belüftungskanal 3 hin, sobald innerhalb eines Fluidspeichers, mit dem die Tropfkammer 1 verbunden ist, im Betrieb eines Infusionssystems ein Unterdruck entsteht. Bei einem Überdruck in dem Belüftungskanal 3 hingegen verschließt die Ventilmembran 9 den Durchtritt 8 druckfest und dicht.

Das Ventilbauteil 4 ist mit seinem Gehäusekörper 6, der elastisch flexiblen Ventilmembran 9 und dem Trägerelement 7 als einstückiges Mehrkomponentenspritzgussbauteil aus Kunststoffmaterialien hergestellt. Dabei ist der Gehäusekörper 6 als harte Bauteilkomponente aus einem thermoplastischen Kunststoffmaterial hergestellt, wohingegen die Ventilmembran 9 und das einstückig mit der Ventilmembran 9 gestaltete Trägerelement 7 als weiche Bauteilkomponente aus einem Elastomermaterial, vorliegend aus einem UV-härtenden Elastomermaterial, nämlich einem UV-härtenden Silikon, hergestellt sind. Der Bakterienfilter 5 wird nach Herstellung des Ventilbauteils 4 eingebracht.

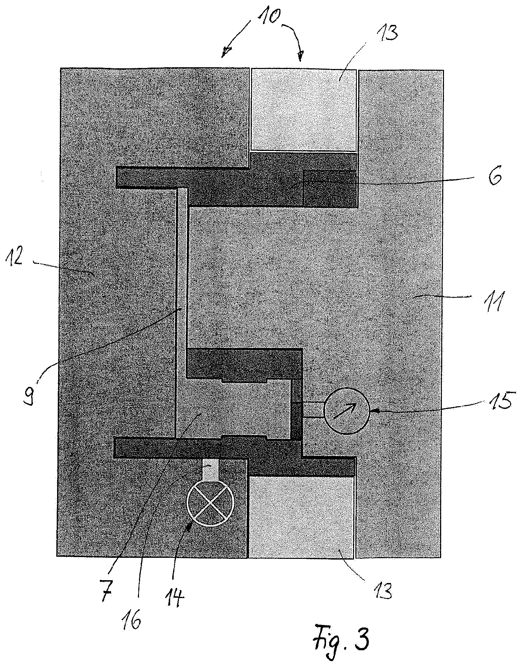

Zur Herstellung des Ventilbauteils 4 gemäß Fig. 2 aus einem thermoplastischen Kunststoffmaterial für den Gehäusekörper 6 einerseits und dem UV-härtenden Elastomermaterial für die Ventilmembran 9 und das Trägerelement 7 andererseits ist ein Kunststoffspritzgusswerkzeug gemäß Fig. 3 vorgesehen. Das Kunststoffspritzgusswerkzeug weist eine Werkzeugform 10 auf, die aus mehreren Formteilen 11 bis 13 zusammengesetzt ist, die im zusammengefügten Zustand entsprechende Kavitäten für die Herstellung der Bauteilkomponenten des Ventilbauteils 4 umgrenzen. Dabei bilden die Formteile 11 und 12 Konturplatten, wohingegen die Formteile 13 Indexplatten definieren. Hinter der Werkzeugform ist wenigstens ein Druckaufnehmer 15 integriert, der zur Prozessüberwachung dient. Zudem ist den Kavitäten, die für die Formung der Ventilmembran 9 und des Trägerelementes 7 vorgesehen sind, eine UV-Lichtquelle 14 mit einem Lichtleiter 16 zugeordnet, wobei der Lichtleiter 16 an entsprechende Innenwandungsabschnitte der Kavitäten angrenzt, um in die Kavitäten eine gewünschte UV-Lichtstrahlung zu bringen.

In einem ersten Prozessvorgang wird zunächst der thermoplastische Kunststoff eingebracht und bis zur Erstarrung abgekühlt, der zur Herstellung des Gehäusekörpers 6 dient. Anschließend wird das UV-härtende Silikon in fließfähiger Form in die verbleibenden Kavitäten eingebracht, wo es die Form des Trägerelements 7 und der Ventilmembran 9 einnimmt. Durch UV-Bestrahlung mittels der UV-LED 14 und des Lichtleiters 16 härtet das Silikonmaterial aus, wodurch sich die gewünschte Verbindung mit dem thermoplastischen Kunststoffmaterial des Gehäusekörpers 6 ergibt, diese kann sowohl stoff-, als auch form- oder kraftschlüssig erfolgen. Als thermoplastische Kunststoffmaterialien für den Gehäusekörper 6 werden Standardthermoplaste wie ABS, PS, PC oder Polyolefine eingesetzt.

## Patentansprüche

1. Kunststoffspritzgusswerkzeug zur Herstellung eines Mehrkomponentenspritzgussbauteils mit einer harten Bauteilkomponente aus einem thermoplastischen Kunststoff, sowie mit einer weichen Bauteilkomponente aus einem Elastomermaterial, wobei die weiche Bauteilkomponente aus einem UV-härtenden Elastomermaterial, insbesondere eine Silikon-Elastomerkomponente, hergestellt ist, die mit der harten Bauteilkomponente aus thermoplastischem Kunststoff verbunden ist, wobei das Mehrkomponentenspritzgussbauteil als Ventilbauteil (4) für den Einsatz bei medizinischen Infusionssystemkomponenten gestaltet ist, und wobei das Ventilbauteil (4) mit einem formstabilen Gehäusekörper (6) aus einem thermoplastischen Kunststoff sowie mit einer elastisch flexiblen Ventilkomponente (9) versehen ist, die aus dem UV-härtenden Elastomermaterial hergestellt ist, sowie mit einer Werkzeugform (10), die einen Zuführanschluss für eine thermoplastische Kunststoffkomponente sowie einen Zuführanschluss für eine UV-härtende Elastomerkomponente, insbesondere eine Silikon-Elastomerkomponente, aufweist, wobei in der Werkzeugform (10) eine UV-Lichtquelle (14) zum UV-Aushärten der Elastomerkomponente integriert ist, **dadurch gekennzeichnet, dass** die Werkzeugform (10) zur Herstellung des Ventilbauteils (4) gestaltet ist.

2. Kunststoffspritzgusswerkzeug nach Anspruch 1, **dadurch gekennzeichnet, dass** die UV-Lichtquelle eine LED-Anordnung (14) mit einem UV-Strahlungsbereich umfasst.

3. Kunststoffspritzgusswerkzeug nach Anspruch 2, **dadurch gekennzeichnet, dass** die UV-Lichtquelle eine Lichtleiteranordnung (16), insbesondere zur Leitung der UV-Lichtstrahlung der LED-Anordnung, umfasst.

## Claims

1. Plastic injection moulding tool for manufacturing a multi-component injection moulding part including a hard part component made of a thermoplastic synthetic material and including a soft part component made of an elastomer material, wherein the soft part component is produced of a UV-curable elastomer material, in particular a silicone elastomer component, which is connected to the hard part component made of thermoplastic synthetic material, wherein the multi-component injection moulding part is configured as a valve assembly part (4) for use in medical infusion system components, and wherein the valve assembly part (4) is provided with a dimensionally stable housing body (6) made of a thermoplastic synthetic material and with an elastically flexible valve component (9) which is produced from the UV-curable elastomer material, and including a tool mould (10) which has a supply connector for a thermoplastic synthetic material component and a supply connector for a UV-curable elastomer component, in particular a silicone elastomer component, wherein a UV light source (14) for UV curing of the elastomer component is integrated in the tool mould (10), **characterized in that** the tool mould (10) is configured for manufacturing the valve assembly part (4).

2. Plastic injection moulding tool according to claim 1, **characterized in that** the UV light source comprises an LED array (14) with a UV radiation range.

3. Plastic injection moulding tool according to claim 2, **characterized in that** the UV light source comprises a light conductor arrangement (16), in particular for conducting the UV light radiation of the LED array.

## Revendications

1. Outil de moulage par injection de plastique pour la fabrication d'un élément moulé par injection à plusieurs composants, comprenant un composant d'élément dur en plastique thermoplastique et un composant d'élément souple en matériau élastomère, le composant d'élément souple étant fabriqué à partir d'un matériau élastomère durcissant aux UV, en particulier un composant élastomère à base de silicone qui est connecté au composant d'élément dur en plastique thermoplastique, l'élément moulé par injection à plusieurs composants étant réalisé sous forme d'élément de soupape (4) pour l'utilisation dans des composants de systèmes de perfusion médicaux et l'élément de soupape (4) étant pourvu d'un corps de boîtier de forme stable (6) en plastique thermoplastique ainsi que d'un composant de soupape élastiquement flexible (9) qui est fabriqué à partir d'un matériau élastomère durcissant aux UV, et avec un moule d'outil (10) qui présente un raccord d'alimentation pour un composant en plastique thermoplastique ainsi qu'un raccord d'alimentation pour un composant élastomère durcissant aux UV, en particulier un composant en élastomère à base de silicone, une source de lumière UV (14) pour le durcissement aux UV du composant élastomère étant intégrée dans le moule d'outil (10), **caractérisé en ce que** le moule d'outil (10) est configuré pour la fabrication de l'élément de soupape (4).

2. Outil de moulage par injection de plastique selon la revendication 1, **caractérisé en ce que** la source de lumière UV comprend un agencement de DEL (14) avec une plage de rayonnement UV.

3. Outil de moulage par injection de plastique selon la revendication 2, **caractérisé en ce que** la source de lumière UV comprend un agencement de conducteur de lumière (16), en particulier pour conduire le rayonnement de lumière UV de l'agencement de DEL.
